# EUROPEAN PATENT APPLICATION

(11) **EP 4 769 429 A1**
(43) Date of publication of application: **01.07.2026**
(21) Application number: 25222334.2
(22) Date of filing: 10.12.2025
(51) Int. Cl.: G16H 40/67

(54) **USER RECOGNITION IN A RPM SYSTEM**

(30) Priority: 26.12.2024 FR 2415305
(71) Applicant: Withings, 92130 Issy-Les-Moulineaux (FR)
(72) Inventor: Attia, Samuel, Issy-les-Moulineaux (FR); Houlbrèque, Vincent, Issy-les-Moulineaux (FR)
(74) Representative: Withings IP

(57) **Abstract**

The disclosure relates to a system including a physiological measurement device (the device) and a server with a user profile, wherein the device generates physiological data and recognition data, and the recognition data are used to compute a recognition score to determine whether the person using the device is the user of the user profile or not. Based on the recognition score, actions are taken.

## Description

### Technical field

The invention relates to the field of recognizing a user performing health-related measurements by a physiological measurement device and, more precisely, the attribution of those health-related measurements to a specific user. This recognition is useful in the field of remote patient monitoring (RPM) and RPM systems.

### Background of the invention

Electronic body scales usually use weight to recognize a user among a plurality of user profiles. This recognition is usually carried out at the beginning of a measurement session. Either the user is recognized, and the measured data are attributed to the user (on the device and on a remote device, such as a phone or a server), or the user is not recognized, and the measured data is not attributed to the user. Most of the time, a guest mode is implemented so that a registered user may still access the non-attributed data.

However, with the development of remote patient monitoring (RPM) and cellular devices, the user has fewer and fewer control of his or her own devices and the data management flow. For example, with a cellular body scale, which is pre-configured for a specific user, all data coming from that scale may automatically be attributed to the user. This may cause data belonging to someone else being attributed to the user and thus undermining the efficiency of the RPM

### Summary of the disclosure

A goal of the invention is to provide a server, a RPM system, and methods associated therewhith which does not present the limitations mentioned previously.

In an aspect, the disclosure relates to a server storing a user profile associated with a user, and configured to exchange data with a physiological measurement device. The physiological measurement device may be configured so that every data generated by the physiological measurement device is systematically associated with the user profile of the server.

The server is configured to receive from the physiological measurement device physiological data generated by the physiological measurement device, wherein at the server the physiological data are associated with the user profile. The server is further configured to receive from the physiological measurement device recognition data associated with the physiological data. The recognition data are data used to determine whether the physiological data were generated by the user of the user profile.

Based at least on the recognition data, the server is configured to compute a recognition score based. This recognition score is then used to manage the physiological data.

For example, managing may include attaching the recognition score to the physiological data so that a remote monitoring interface may visually flag the physiological data or so that another server may delete the physiological data (especially when the recognition score reflects that the physiological data were not generated by the user of the user profile). Managing may include removing the physiological data from the user profile (especially when the recognition score reflects that the physiological data were not generated by the user of the user profile). Managing may include triggering a user request to confirm or reject the physiological data (especially when the recognition score reflects that there is a doubt about the person who generated the physiological data). Managing may include generating instructions to delete the physiological data of the user profile based on the recognition score, either at another server or at the physiological measurement device.

The recognition data may be taken from the physiological data. In an embodiment, the physiological data is a user weight, and the recognition data are the user weight as well.

According to this aspect, a system is also disclosed, wherein the system is notably a RPM system, and includes the server as presented above and a physiological measurement device (the device). The server and the device are configured to exchange data through a communication network.

Particularly, the device is a mono-user device. This means that the device is configured so that every data generated by the device is systematically associated with the user profile of the server.

The additional features provide further implementation details.

In an embodiment, the user profile includes reference data, and the recognition score is computed based on a comparison between the reference data and the recognition data.

In an embodiment, computing the recognition score takes into account: a value difference between a value of the generated recognition data and a value of the reference data, and a time difference between a timestamp of the generated recognition data and a timestamp of the reference data. This allows taking into account the variation of physiological data over time.

In an embodiment, a reference score is attached to the reference data and computing the recognition score takes into account the reference score, so that the recognition score can only be strictly lower than the reference score.

In an embodiment, the server or system is configured to, in response to determining that the recognition score is above a certainty threshold, update the reference data with the generated recognition data. The reference data are then up-to-date, which allows a recognition process as good as it can be.

In an embodiment, the system is further configured to, in response to determine that the recognition score is below an uncertainty threshold, remove the generated physiological data and the generated recognition data from the user profile.

In an embodiment, the user profile includes a notification channel to the user and the system is further configured to, in response to determining that the recognition score is below the certainty threshold, trigger by the server a request on the notification channel to confirm or reject that the received physiological data and the recognition data are to be attributed to the user profile.

The server, then, in response to receiving the confirmation, updates the reference data of the user profile with the generated recognition data, and/or in response to receiving the rejection, removes the generated recognition data and the generated physiological data of the user profile.

In an embodiment, the server is further configured to, in response to determining that the recognition score is below a certainty threshold and above an uncertainty threshold and in the absence of receiving a confirmation or a rejection within a predetermined time period, updating the reference data with the obtained recognition data, or/and in response to determining that the recognition score is below the uncertainty threshold and in the absence of receiving a confirmation or a rejection within a predetermined time period, deleting the recognition data and the physiological data of the user profile.

In an embodiment, the server includes a first server and a second server, and the user profile includes a first user profile stored at the first server and a second user profile at the second server, wherein the second user profile includes at least the first user profile, wherein the second user profile and the first user profile are associated with the same user, and the second server is connected to a remote monitoring interface. The system is further configured to send by the first server the physiological data with the recognition score to the second server, and remove the generated physiological data of the second user profile based on the recognition score.

In particular, determining and removing are performed by the second server, and the removing is done on the second user profile. The notification channel is included in the second user profile, and triggering is performed by the second server.

In an embodiment, the system is further configured to send to the physiological measurement device instructions to delete the generated physiological data and the generated recognition data.

The notification channel may involve a user terminal, such a user mobile phone. In an embodiment, the user cannot manage the physiological data from his or her user terminal. This avoids deleting or data alteration by the user.

In an embodiment, the system further comprises initializing the reference data of the user profile, wherein initializing is done by:
- sending through a notification channel a request to use the physiological measurement device during a time window and receiving recognition data during that time window, and setting the reference data as the recognition data,
- initially providing the reference data by a third party,
- manually inputting the reference data.

In an embodiment, the system further comprises a remote monitoring interface, wherein the remote monitoring interface has a specific visual feature for the physiological data with a recognition score under a certainty threshold.

In an embodiment, the recognition data are taken from the physiological data. This allows to reduce the effort and constraint on the user. Alternatively, the recognition data are different from the physiological data. This allows to have recognition data better adapted for recognition than physiological data, notably in the case in which the physiological data are not appropriate to recognize a user.

In an embodiment, the recognition data include at least one of weight data and impedance data.

According to this aspect, the disclosure also relates to a method for remotely monitor patients, using a system as disclosed above, wherein the method comprises:
generating physiological data by the physiological measurement device,
generating recognition data by the physiological measurement device, wherein the recognition data are associated with the physiological data,
   - receiving by the server the generated recognition data and the generated physiological data, wherein the physiological data and the recognition data are attributed to the user profile of the server,
   - computing a recognition score based at least one the generated recognition data, the recognition data being used to determine whether the physiological data were generated by the user of the user profile,
   - managing the generated physiological data based at least one the recognition score.

According to another aspect, the disclosure also relates to a physiological measurement device (the device) storing a user profile associated with a user, and configured to exchange data with a server. The device may be configured so that every data generated by the device is systematically associated with the user profile of the device and thus of the server.

The device is configured to generate physiological data, wherein at the server the physiological data are associated with the user profile, and to generate recognition data associated with the physiological data. The recognition data are data used to determine whether the physiological data were generated by the user of the user profile.

Based at least on the recognition data, the device is configured to compute a recognition score based. The recognition score may be attached to the physiological data. The recognition score and the physiological data are then sent to a server. This recognition score is then used to manage the physiological data, at the device, and/or at a server.

Managing the physiological data may be as disclosed previously.

The device may notably be a body scale.

This other aspect also relates to a method for remotely monitoring a patient using the device previously disclosed.

The additional features provided above are also applicable inasmuch as they relate to the server, except that the recognition score is not computed by the server.

### Description of the drawings

Other features will be visible on the following non limitative drawings:
[Fig. 1] Figure 1 shows a remote patient monitoring, RPM, system, according to an embodiment of the disclosure,
[Fig. 2] Figure 2 shows a device (a body scale)according to an embodiment of the disclosure,
[Fig. 3] Figure 2 shows a remote monitoring interface run on a monitoring device according to an embodiment of the disclosure,
[Fig. 4] Figure 4 shows two different server configurations according to variants of the disclosure,
[Fig. 5] Figure 5 shows physiological data (user weight data) and recognition data (user weight data as well) taken at different times and with different values,
[Fig. 6] Figure 6 shows two examples for computing a recognition score,
[Fig. 7] Figure 7 shows an example of a display mode with notification levels on the remote monitoring interface according to an embodiment of the disclosure,

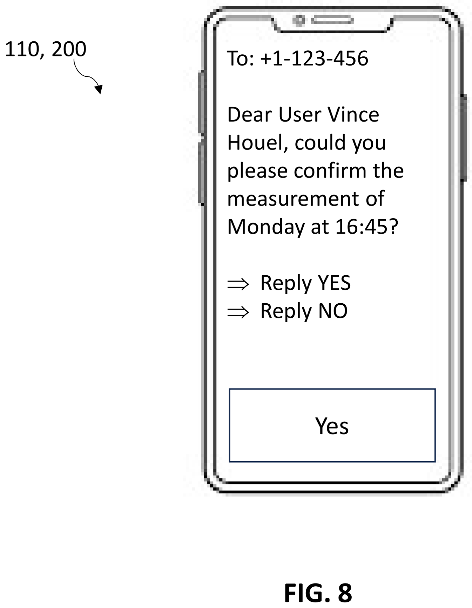

[Fig. 8] Figure 8 shows an example of a user request via the notification channel, according to an embodiment of the disclosure,

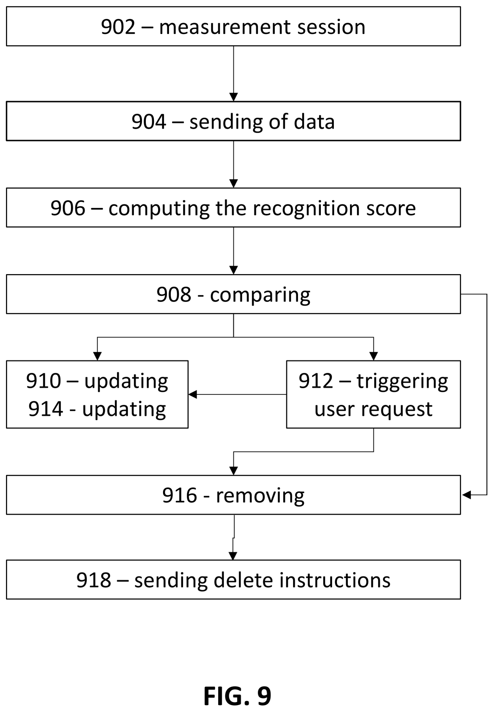

[Fig. 9] Figure 9 shows a RPM method performed by a RPM system, according to an embodiment of the disclosure.

### Detailed description

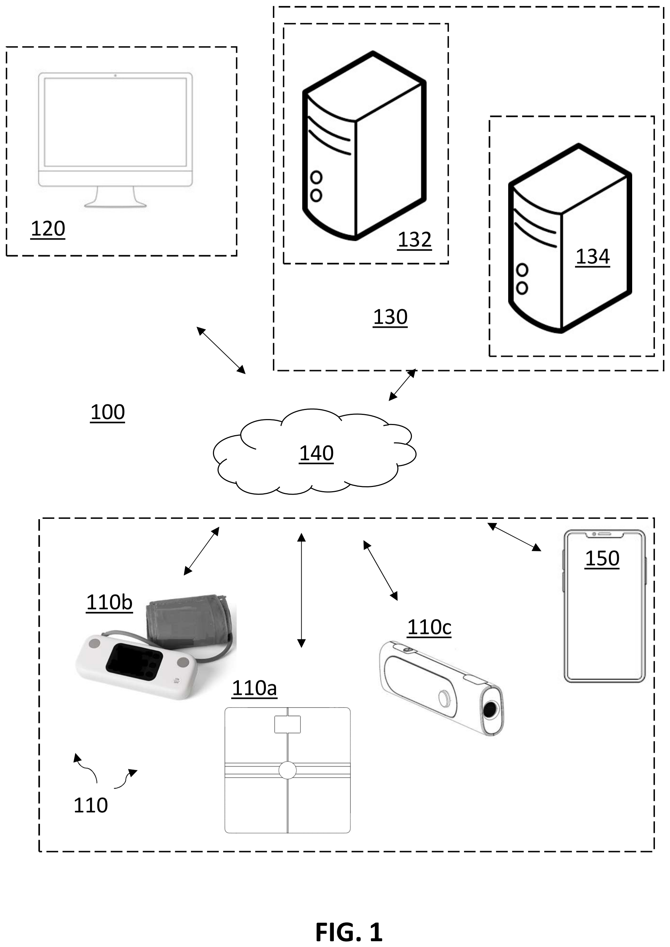

Figure 1 illustrates a remote patient monitoring system 100, called RPM system 100. The RPM system includes at least one physiological measurement device 110, called device 110 (here a body scale 110a, a blood pressure monitor 110b, and/or a multiscope 110c (such as disclosed in EP24217550 or devices called BeamO^{™}), but could comprise other devices, such as those disclosed in EP4386383 to Withings^{™}, or devices U-Scan^{™} developed by Withings^{™}), a remote monitoring interface 120, a server 130, which may comprise a first server 132 and a second server 134 and a communication network 140. The RPM system 100 may comprise also a user terminal 150, such as mobile device, like a smartphone, a smartwatch, or a laptop. The remote monitoring interface 120 is configured to run on a remote monitoring device (not shown on Figure 1), which is remotely arranged from the device 110.

The server 130 is interfacing between the device 110 and the remote monitoring interface 120.

In a one-server embodiment, the server 130 is a single functional entity. In that case, the device 110, the server 130 and the remote monitoring interface 120 may be provided by a same provider.

In a dual-server embodiment, the server 130 includes the first server 132 which is interfacing between the second server 134 and the device 110, while the second server 134 is interfacing between the first server 132 and the remote monitoring interface 120. In that case, the device 110 and the first server 132 are generally provided by a first provider, while the second server 134 and the remote monitoring interface are provided by at least one second provider. The first server 132 and the second server 134 may communicate data through one or more API (application programming interface) for example. The first server 132 receives all data coming from the device 110 and sends all of them or a part of them to the second server 134. The remote monitoring interface 120 may only access data from the second server 134. In the dual server embodiment, the actions of the present description may be carried out by the first server 132 or the second server 134.

The RPM system 100 is used by various people, who are defined as follows: the user of the device 110 is referred to as a patient and the user of the monitoring device 120 is referred to as a professional, since he or she is a healthcare professional, such as a nurse or a physician. The professional and the user in a RPM system are usually not in the same room, but usually at their respective work or living locations, except for a specific appointment, such as a follow checkup.

In a typical situation, the device 110 generates physiological data from the patient during a measurement session and sends the physiological data to the server 130 via the communication network 140, then the remote monitoring interface may access the physiological data from the server 130 (or any data obtained from the physiological) via the communication network 140.

The RPM system 100 is aimed at gathering physiological data on the patient, via the device 110, and at providing them to the professional, via the remote monitoring interface 120.

The remote monitoring interface 120, the monitoring device 110 and the server 130 (including the first server 132 and the second server 134 if applicable) all exchange data using the communication network 140, which may be a hybrid network (ethernet, Wi-Fi, 3G, 4G, 5G, etc.).

### The device 110

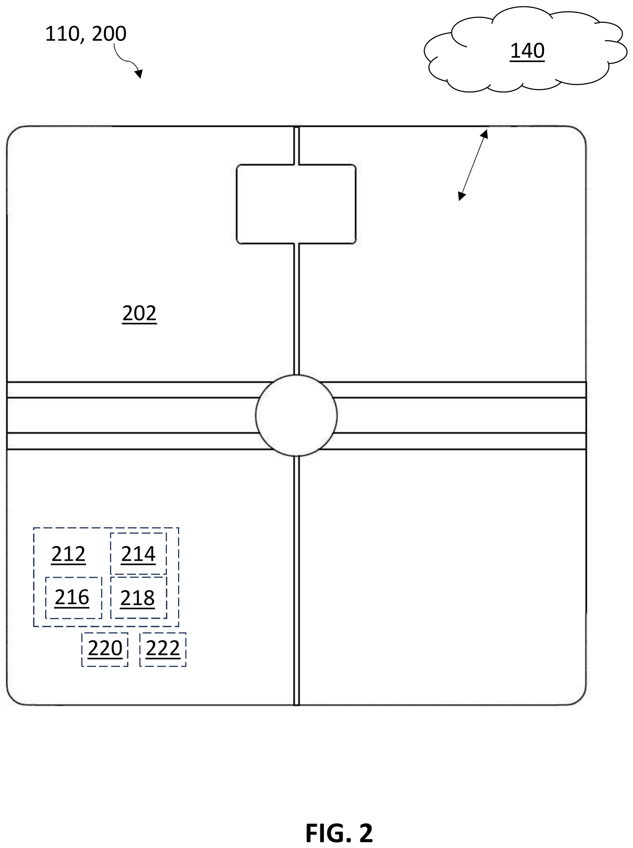

Figure 2 illustrates a device 200 corresponding to the body scale 110a of Figure 1. The device 200 includes at least one physiological sensor assembly 202, such as a weight sensor, a ECG (electrocardiogram) sensor, a PPG (photoplethysmogram) sensor, an IPG (impedanceplethysmogram) sensor, etc.

The device 200 notably generates physiological data using the physiological assembly 202.

As schematically shown on Figure 2, the device 200 includes a control unit 212, comprising a processor 214, a memory 216 and a I/O (in/out) interface 218. The memory 216, which includes storage, is configured to store instructions, which, when executed by the processor 214, allows the device 200 to notably generate physiological data of a user, and send the physiological data. The I/O interface 218 manages the interactions of the control unit 212 with the other components. A communication module 220 enables the device 200 to exchange data, typically in a wireless manner with the communications network 140. A battery 222 stores energy to allow the device 200 to work without being electrically plugged.

The device 200 may store a history of the last measurement sessions, so that it can display information related to the evolution of physiological data.

In an embodiment the communication module 220 is a cellular module capable of exchanging data on a cellular network of the communication network, so that no user terminal 150 is required to forward any data generated by the device 200.

In an embodiment, the device 200 is multi-user, meaning that it may be shared by several users. To that end, each user has a user profile. Data generated for a same user is associated with the same user profile. In that case, a user recognition may be carried out by the device 200. However, in case of a misrecognition, the data are sent to the wrong registered user on the server 130 and will populate the wrong user profile with alien physiological data. This pitfall is paramount to avoid in an RPM system since medical actions may then be taken by the professional based on the physiological data.

In another embodiment, the device 200 is mono-user, meaning that any data generated by the device 200 is attributed to a unique user profile, regardless of who actually used the device 200. This configuration creates difficulties since another person may use the device 200 and the generated data will be attributed to the unique user profile and thus populate the user profile with alien physiological data. This pitfall is paramount to avoid in an RPM system since medical actions may then be taken by the professional based on the physiological data.

When the user uses the device 110200, he or she starts a measurement session, during which physiological data of the user are to be generated, as well as recognition data. Therefore, unless said otherwise, any recognition data are generated along physiological data and are associated with them.

The physiological measurement device 110 is an electronic device.

### The remote monitoring interface 120

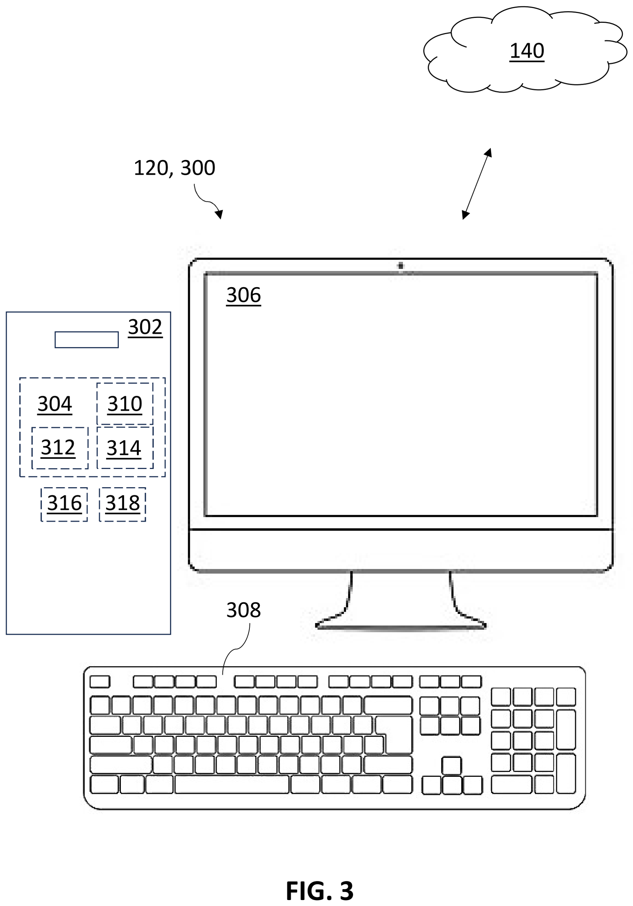

**Figure 3** illustrates a remote monitoring interface 300 corresponding to the remote monitoring interface 120 of Figure 1. The remote monitoring device interface 300 can be run by a monitoring device 302 using a control unit 304 capable of processing data, including receiving data from the server 130 and displaying data. The remote monitoring interface 300 is configured for permitting an interaction with the professional. To that end the remote monitoring interface 300 may include a display 306, such as a screen, and a hardware input 308, such as a keyboard and/or a mouse, or a trackpad. The remote monitoring device 302 is typically a personal computer (e.g., a laptop).

The remote monitoring interface 300 is typically a software-based interface, which can be deployed as an app, for example accessible on an Appstore^{™}, or a webapp or a web page accessible from a navigator. The remote monitoring interface 300 is generally provided as a SaaS (software as a service).

As schematically shown on Figure 3, the control unit 304 comprises a processor 310, a memory 312 and an I/O (in/out) interface 314. The memory 312, which includes storage, is configured to store instructions, which, when executed by the process, allows the monitoring device 302 to notably display information on the display 306 and receive user input via the hardware input 308. The I/O interface 314 manages the interactions of the control unit 302 with the other components. A communication module 316 enables the monitoring device 302 to exchange data with the communication network 140. A battery 318 may store energy to allow the monitoring device to work without being electrically plugged.

To qualify as a RPM system 100, the remote monitoring interface 120, 300 is generally not activated on the device 110 or the user terminal 140 of the user, but only on the monitoring device 302 of the professional.

The remote monitoring interface 120, 300 gives access to the professional to data from various user profiles, notably (and only) the user profiles of the users which are under a RPM program by the professional.

### The server 130

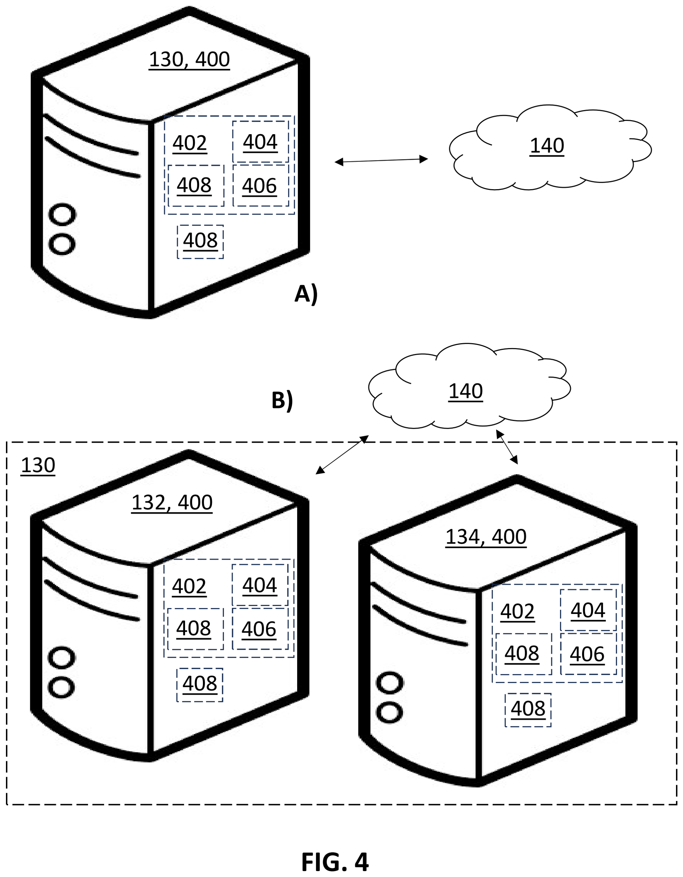

Figure 4 illustrates two configurations A) and B), for the one-server embodiment and the dual-server embodiment. In configuration A), the server 130 of Figure 1 includes a server 400. In configuration B), the server 130 includes a first server 132 and the second server 134 of Figure 1, whicheach comprise a server 400

The server(s) 400 are a remote hardware unit configured to receive, store and process data, for example received from the device 110 or from another server 400, and to send the physiological data or any related data to the remote monitoring interface 120 or to another server 400. Conversely, the server 400 is also configured to receive, store and process data received from the remote monitoring interface 120 and to send the data or any related data. The server 130 is typically remotely located from the device 110 and the remote monitoring interface 120. The server 130 may be located in a datacenter, for example a datacenter provided by a datacenter provider or by the device provided or by the hospital data center of the professional.

The server 400 store user profiles (with all the associated data) in a centralized manner and acts as a cloud server. In configuration B), the first server 132 may store a first user profile and the second server 134 may store a second user profile. Those profiles may be synchronized from time to time. The second user profile may be included in the first user profile.

As schematically shown on Figure 4, any server 400 includes a control unit 402, comprising a processor 404, a memory 406 and a I/O (in/out) interface 408. The memory 406, which includes storage, is configured to store instructions, which, when executed by the process, allows the server to perform method as disclosed in the description. The I/O interface 408 manages the interactions of the control unit 402 with the other components. A communication module 408 enables the device 200 to exchange data, typically in a wired manner with the communications network 140.

Each of the servers 400 may technically include a plurality of servers.

In the rest of the description, the repartition of task between the first server 132 and the second server 134 will not be detailed and they will be referred to as the server 130. A specific section at the end deals with the repartition of action.

### General presentation

In the rest of the description, it will be considered that the device 110 is mono-user, with the user having a user profile in the server 130 or that the device 110 is multi-user and one of the users has a user profile in the server 130 (other users may have their own user profiles).

The device 110 is configured to generate physiological data during a measurement session, as described above. Typically, the physiological data include weight data. Additionally, they include impedance data (such as the fat mass, water mass, bone mass, etc.), ECG data, PPG data, etc. The physiological data have a timestamp, which corresponds to the time at which the physiological data were generated. The physiological data are representative of a state of the user and have a medical or wellness purpose. Therefore the physiological are data of interest for RPM.

The device 110 is also configured to generate recognition data during the same measurement session. The purpose of the recognition data is to determine whether the physiological data were generated by the user of the user profile of the server 130. The acquisition of recognition data must not be demanding or challenging for the user, which is why they are acquired by the device 110. As the recognition data are generated during a measurement session for generating physiological data, their generation does not request an additional input or effort from the user.

In an embodiment, the recognition data may be taken from the physiological data. For example, the physiological data include weight data (such as the weight of the user) and the recognition include or are the weight data (such as the weight of the user as well). For example, the physiological data may include impedance data and the recognition data include impedance data, with or without weight data. Other physiological data may be used, such as the ECG and the recognition data may include the ECG.

Alternatively, the recognition data are distinct from the physiological data. For example, the recognition data are weight data which does not represent the weight of the user, such as a weight evolution representing a weigh pattern at the scale by the user (leaning to the left or right, or stepping with the left foot rather than the right foot).

The server 130 receives the generated recognition data and the generated physiological data, from the measurement session, which are attributed to the user profile, either because the device 110 is mono-user or because the user profile was recognized on the device 110.

The server 130, using at least the recognition data, computes a recognition score representative of a likelihood that the user associated with the user account of the server 130 is at the origin of the measurement session, and thus of the physiological data and the recognition data. This recognition score is then used to manage the generated physiological data, for various purposes, either by the server 130 (this may differ between the one-server embodiment and the dual-server embodiment). Managing may involve attaching the recognition score to the physiological data and sending both of them, deleting the physiological data at the user profile, or generating instructions to delete the physiological data at the user profile or on the device 110. This will be disclosed in more detail later.

### The recognition score

In an embodiment, computing the recognition score is made using reference data, which are stored in the user profile at the server 130. The reference data allows to recognize the user based on the reference data.

The reference data may typically be previously generated recognition data, either the latest generated recognition data or a history of latest generated recognition data, so as to anticipate a trend. As it will be described later, the reference data may be updated with the generated recognition data. This allows the reference data to be up-to-date. This will be described in further detail later.

More precisely, computing the recognition score involves comparing the reference data and the recognition data. This comparison may involve comparing values of the reference data and of the recognition data. However, as recognition data may vary over time, especially when the recognition data are taken from the generated physiological data, the comparison may also involve comparing timestamps of the generated recognition data and of the reference data. In another embodiment, the reference data also has a reference score attached thereto and the computed recognition score takes into account the recognition score of the reference data (notably it is calculated from the recognition score).

The computed recognition score is then compared to at least one threshold. In particular, a certainty threshold Ct is defined, which characterizes the fact that for a recognition score above the certainty threshold, it is assumed that the user of the user profile did take the measurement. In a similar manner, an uncertainty threshold Ut may be defined, which characterizes the fact that for a recognition score below the uncertainty threshold, it is assumed that the user of the user profile did not take the measurement. More details will be given later.

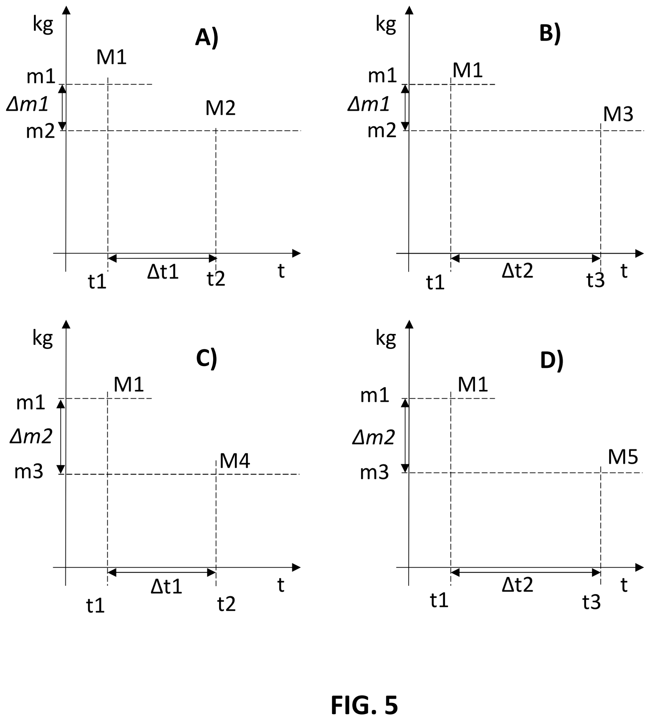

Figure 5 illustrates different examples A), B), C) and D) in which the recognition data are weight data and more precisely the weight of the user of the user profile and the device 110 is a body scale.

The A), B), C) and D) examples illustrate four different situations. In all of them, measurement M1 represents the weight m1 (which is the value of the weight data) of a user at time t1 (which is the timestamp of weight data). The reference data were set to be m1. There are three situations: in situation A), at t2, a user steps on the body scale 110a for a measurement M2 of value m2, in situation B), at t3, a user steps on the body scale 110a for a measurement M3 of value m2, C), at t2, a user steps on the body scale 110a for a measurement M4 of value m3, D), at t4, a user steps on the body scale 110a for a measurement M5 of value m3.

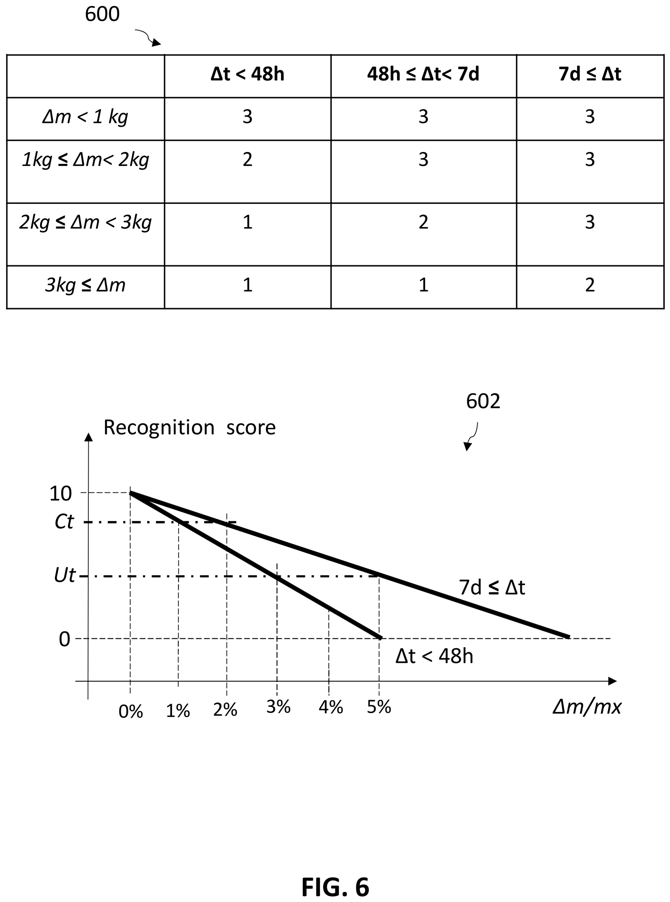

Timestamp t3 is later in time than t2 and m3 is lower than m2 in kg (represented by Δm1, Δm2, Δt1, Δt2 on figure 6).

Assuming that M1 was generated by the user of the user profile. Then the recognition score is defined so that, for a specific time difference Δt, the smaller the weight difference, the higher the recognition score, and the shorter the time difference the higher the score. Typically, thresholds are set up for several ranges of time differences. We call "function" the mathematical relation between the inputs (reference data, recognition data, and if applicable, the reference score) and the computed recognition score.

Table 600 on Figure 6 illustrates a basic example of a function which provides values for recognition score, based on different value differences (Δm) and time differences (Δt). In this example, the recognition score may take three values (1, 2, 3). Instead of absolute value differences, the table 600 could use relative value differences (by computing Δm/mx, where mx is the value of the generated weight). The certainty threshold Ct may be here 2,5 and the uncertainty threshold may be 1,5.

The recognition score may be computed in a more refined way, with more values. For example, instead of thresholds as in table 600, the function defining the recognition score may be continuous function of Δm, wherein the function itself depends on Δt. Let mx be the weight of the measurement session.

Graph 602 on figure 6 shows a more refined example as described previously. Here the function is a linear function, whose slope depends on Δt. The slope defines the recognition score in function of Δm. Two slopes are given, for Δt < 48h (measurements close to each other in time) and for 7d ≤ Δt (d standing for days) (measurements spaced from each other). The certainty threshold Ct is reached for small weight variation for close measurements in time than for more spaced apart measurement in time (here 1% v. 2%). The uncertainty threshold Ut here is 3% for close measurements in time while it is 5% for more spaced apart measurement in time. The recognition score may take eleven values (from 0 to 10).

In another embodiment, the recognition score is computed based on the recognition score of the reference data (called reference score). For example, instead of having table 600 or the graph 700 which computes the recognition score in absolute, the function may provide an amount to subtract to the reference score, so as to compute the recognition score. In this case, the smallest amount to subtract is not zero, so that even recognition data identical to the reference score are not granted the highest recognition score. This way, even regular recognition data variations (e.g., small weight variations) will still lead the recognition score to go below the certainty threshold Ct at some point in time.

In this embodiment, the inputs of the function are the reference data, the recognition data, and the reference score and the output is the computed recognition score.

The function may be even more complex and involve for example a machine learning algorithm, which takes into account a history of recognition data (values and timestamps) to generate a more precise recognition score

### Actions to be taken in function of the recognition score

The server 130 stores the reference data, receives the generated physiological data and the generated recognition data, computes the recognition score. Then based on the recognition score, the server 130 takes different actions. The actions to be taken using the recognition score depends on the overall architecture of the RPM system. Some actions are carried out by the server 130, some by the remote interface monitoring 120 and some by the device 110. In the dual server architecture, the actions may be carried out by the first sever 132 and/or the second server 134.

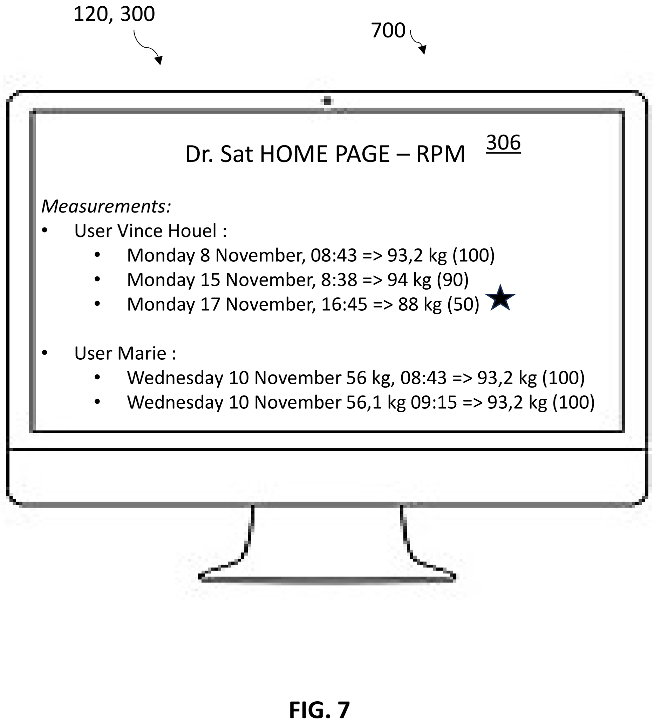

In an embodiment, the server 130 attaches the recognition score to the physiological data and the remote monitoring interface 120 displays the physiological data taking into account the recognition score. For example, a low recognition score (lower than the certainty threshold Ct or lower than the uncertainty threshold Ut) triggers an alert on the physiological data for the professional. Figure 7 shows an interface 700 on the remote monitoring interface 120, with a specific visual feature for the physiological data with a low recognition score, such as lower than the certainty threshold Ct (here a star, for the weight measurement of Monday 17 November of user profile Vince Houel, followed by Dr. Sat on the remote monitoring interface 120 - all names and data are made up for the illustration), although the recognition score is not displayed as such on this example.

The server 130 stores the certainty threshold Ct. In an embodiment, in response to determining that the recognition score is above the certainty threshold Ct, the server 130 updates the reference data with the generated recognition data. As the physiological data were already attributed to the user profile, the server 130 does nothing in particular about them, so as to actually keep them in the user profile. For example, in relation with table 600 of Figure 6, the certainty threshold Ct is equal to 2,5.

Additionally, or alternatively, the RPM system, in some situations that require clarity, may trigger a user request. The goal of the RPM system is to streamline the measurement process and to avoid any pain point. Any wrong data at the remote monitoring interface is an issue for the professional and the user of the user profile. Any interaction with the user which is not a medical interaction becomes a pain point which may disengage the user. The recognition score is aimed at finding a good balance between these two pain points.

The user profile at server 130 may store a notification channel, which allows the server 130 to communicate with the user. For example, the notification channel may exchange data with the user terminal 150 of the user. The notification channel may include a phone number, an app, etc.

In response to determining that the recognition score is below the certainty threshold Ct and for instance above the uncertainty threshold Ut (therefore between Ct and Ut),, the server may thus trigger a user request on the notification channel to confirm or reject that the measurement session was made by the user of the user profile, i.e. that the generated physiological data and the recognition data belongs to the user. To be efficient, the request is to be sent a few minutes or hours after the measurement session.

The request is typically of the yes or no question type, such as: *"was it you who took a measurement on [date and time]?"* (since the user terminal 150 is generally personal) or *"was it [name of the user of the user profile] who took a measurement on [date and time]?".* Figure 7 shows an example of a user request 700 on the user terminal 150, which is via a SMS on a phone.

When the notification channel comprises a phone number (which is almost systematically needed in RPM system, since the professional needs to be able to reach out to the user of the user profile), that the server 130 may send out an SMS to that phone number or make an automated phone call. The response may be a "YES" or a "NO" send through text or spoken over the phone. When the notification channel comprises an app, through which the server 130 may push data or questions, the user may directly respond in the app.

The response (confirmation or rejection) is then sent to the server 130 which then takes action accordingly.

If the response is a confirmation that the user who took the measurement is that of the user profile, then the reference data at the server 130 are updated with the generated recognition data. The generated physiological data are kept at the user profile, since they were attributed on the server 130 to the user of the user profile. Depending on the embodiment, the recognition score (reference score) of the reference data is updated to the maximum possible, since there has been a confirmation that the recognition data belonged to the user of the user profile. This resets the progressive decrease of the recognition score and thus create a regularly check via the notification channel.

If the response is a rejection that the that the user who took the measurement is that of the user profile, then the generated recognition data and the generated physiological data are removed from the user profile at the server 130. Additionally, the server 130 may send instructions to delete the latest measurement session stored on the device 110. This may be done by attributing a recognition score of the lowest possible value and having the removal performed in response to determining that the recognition score is under a deletion threshold or has the lowest possible value.

This user request may be triggered on the sole condition that the recognition score is below the certainty threshold Ct.

Conversely, the server 130 stores the uncertainty threshold Ut, and recognition score below Ut are assumed to mean that the user of the user profile did not made the measurement session, but it was someone else. Therefore, in an embodiment, in response to determining that the recognition score is below the uncertainty threshold Ut, the server 130 removes the generated recognition data and the generated recognition data from the user profile. The server 130 thus does not display the physiological data at the remote monitoring interface 120, thereby avoids the professional to spend time on wrong data.

However, because, for some patients susceptible of undergoing strong weigh variations in a few day (cardiac compensation or decompensation for instance), it is necessary to monitor quick weight variations. In this embodiment, the physiological data are not deleted but a user request may be triggered as disclosed previously (hence the comparison with the certainty threshold Ct is sufficient).

In case the user does not respond to the user request within a predetermined time period (e.g. 3 days), the server 130 may consider that it is a confirmation (called confirmation by default), to avoid removal of data by default. However, this confirmation by default may be applied only when the recognition score is between the certainty threshold Ct and the uncertainty threshold Ut. Below the uncertainty threshold Ut, the server 130 may consider that an absence of response is a rejection (called rejection by default) and thus operate as disclosed above in case of a rejection.

In some embodiment, the user request is not systematically triggered but only after a plurality of measurement sessions for which the recognition score is under the certainty threshold. Upon confirmation, all the previous physiological data with a recognition score higher than the last one are also confirmed.

### The reference data

As explained previously, the reference data may be updated with the latest recognition data, since it is assumed that the most recent recognition data are more relevant than any previous reference data. In an embodiment disclosed above, the recognition score of the latest recognition data is attributed to the reference data.

In an embodiment, an initialization of the first reference data is performed, by manual input of reference data. This is particularly easy when the reference data is the weight of the user. For example, the RPM process may involve an on-boarding visit in person with the professional or any professional, so that the user may be weighed during the on-boarding visit. The professional then uploads the reference data on the server 130, for example via the remote patient monitoring interface 120.

In another embodiment, the initialization may involve an accompanied generation of reference data. This can take several forms.

In an example, the first measurement session on the device 110 is considered to be taken by the user of the user profile. Therefore the reference data will be taken from that first measurement session. It is fair to assume that, as the device 110 is first set up at the user's home, the user of the user profile is the one setting up the device 110. In an example, the RPM system informs the user to generate reference data by using the device 110 during a certain time window, so that the RPM system knows it is the user of the user profile who generated the reference data.

In those cases, the reference data may be attributed the highest recognition score.

### Methods

The RPM system 100 disclosed above is configured to perform the following methods. Depending on the embodiments, some steps may be performed by the server 130 (one server embodiment) or any of the first server 132 and the second server 134 (dual server embodiment).

At 902, during a same measurement session, the device 110 generates physiological data and recognition data. The recognition data may be taken from the physiological data, to avoid adding constraint to the measurement session. For example the recognition data include weight data.

At 904, the device 110 sends the physiological data and the recognition data through the communication network 140. In a mono-user scale, those data are sent without condition on them to the server 130. This means that if an animal or a kin or a thief uses the device 110, the data will be attributed to the user profile on the server 130. The same applies if the device 110 carries out a wrong internal recognition process. At 906, the server 130 receives them.

At 906, the server 130 computes the recognition score, using the recognition data. The recognition is attached to the physiological data.

At 908, the server 130 determines whether the recognition score is above the certainty threshold Ct or below it. The server 130 may also determine whether the recognition score is above the uncertainty threshold Ut or below it. At 910, in response to determining that the recognition score is above the certainty threshold, the server 130 updates the reference data with the generated recognition data, and, if applicable, update the reference score with the computed recognition score. At 912, in response to determining that recognition score is below the certainty threshold, the server 130 trigger a user request on the notification channel of the user profile, to confirm or reject that the measurement session was generated by the user of the user account at the server. The notification channel uses for example the user terminal 150 of the user.

At 914, in response to receiving the confirmation, the server 130 updates the reference data of the user profile with the generated recognition data and, if applicable, the reference score is updated with the maximum possible value. At 916, in response to receiving the rejection or in response to determining that the recognition score is below the uncertainty threshold Ut, the server 130 removes the generated physiological data from the user profile. The recognition data 130, as they were attached to the generated physiological data, are also removed.

At 918, in response to receiving the rejection or in response to the removal, the server 130 sends instructions to the device 110 to remove the latest physiological data and recognition data of its memory. This makes sure that the user will not see a meaningless visual representation of his or her physiological data on a screen of the device 110.

In an embodiment, the server applies a rejection by default or a confirmation by default disclosed previously.

### One server or dual server configuration.

In a one server configuration for the server 130, all the steps are carried out by the single server 130, as defined previously.

In a dual server embodiment for the server 130, several variants exist.

In a variant, the first server 132 merely forwards data between the device 110 and the second server 132, so that the second server 132 performs most of the steps of the method performed by the server 130.

In a variant, the steps of server 130 which involve a communication with the device 110 are performed by the first server 132 and the other steps are performed by the second server 134. This involves transmission and reception steps between servers that are implicit in the description. In this variant, the first server 132 merely forwards data between the device 110 and the second server 134.

In a variant, the first server 132 computes the score and forwards the physiological data with the score to the second server 134, which performs the rest of the steps. Notably, the second server 134 triggers the notification channel. The second server 134 sends the confirmation to the first server which then updates the reference data and, if applicable, the reference score. As disclosed previously, each server 132, 134 may store a user profile of the user. Synchronization may be done only for data that is used by each server. For example, if the second server includes nutrition data that are not needed by the first server, no synchronization on those data is performed.

In a variant, which does not correspond to the dual server embodiment, one of the servers only communicates with the other server to retrieve the recognition data and the reference data, in order to compute the recognition score and to send it to the other server.

In practice, as already mentioned, the recognition data is the user weight. Therefore, the probability score is attached to the physiological data itself.

### Alternative design

In another embodiment, the recognition score is computed by the physiological measurement device 110 itself, and send to the server 130. As it has been shown, the computation of the recognition score requires reference data. Those reference data may be received from the server or, as they may be previous recognition data, are already stored at the device 110, in a user profile on the device 110. Once received by the server 130, the method is similar to any disclosed in this description, notably the comparison with the thresholds, the user request, etc.

## Claims

1. A remote patient monitoring, RPM, system comprising at least:
- a server (130) storing a user profile associated with a user, and
- a physiological measurement device (110), wherein every data generated by the physiological measurement device (110) is systematically associated with the user profile of the server,
wherein the RPM system is configured to:
- generate physiological data by the physiological measurement device,
- generate recognition data by the physiological measurement device, wherein the recognition data are associated with the physiological data,
- receive by the server the generated recognition data and the generated physiological data, wherein the physiological data and the recognition data are attributed to the user profile of the server,
- compute by the server a recognition score based at least one the generated recognition data, the recognition data being used to determine whether the physiological data were generated by the user of the user profile,
- manage the generated physiological data based at least one the recognition score.

2. The RPM system of claim 1, wherein managing the physiological data includes at least one of: attaching the recognition score to the physiological data, removing the physiological data of the user profile based on the recognition score and/or trigger a user request to confirm or reject the physiological data, and/or generating instructions to delete the physiological data of the user profile based on the recognition score.

3. The RPM system of any of claims 1-2, wherein the user profile includes reference data, and the recognition score is computed based on a comparison between the reference data and the recognition data, wherein for example a reference score is attached to the reference data and computing the recognition score takes into account the reference score, so that the recognition score can only be strictly lower than the reference score.

4. The RPM system of claim 3, wherein computing the recognition score takes into account:
- a value difference between a value of the generated recognition data and a value of the reference data, and
- a time difference between a timestamp of the generated recognition data and a timestamp of the reference data.

5. The RPM system of claim 3 or 4, further configured to:
- in response to determining that the recognition score is above a certainty threshold, update the reference data with the generated recognition data.

6. The RPM system of any of claims 3-5, further comprising initializing the reference data of the user profile, wherein initializing is done by either one of:
- sending through a notification channel a user request to use the physiological measurement device during a time window and receiving recognition data during that time window, and setting the reference data as the recognition data.
- initially providing the reference data by a third party,
- manually inputting the reference data.

7. The RPM system of any of claims 1-6, wherein the user profile includes a notification channel to the user, the system being further configured to:
- in response to determining that the recognition score is below the certainty threshold, trigger by the server a request on the notification channel to confirm or reject that the received physiological data and the recognition data are to be attributed to the user profile,

8. The system of claim 7, further comprising:
- in response to receiving the confirmation, update the reference data of the user profile with the generated recognition data, and/or
- in response to receiving the rejection, remove the generated recognition data and the generated physiological data of the user profile.

9. The RPM system of any of claims 1-8, wherein the server includes a first server (132) and a second server (134), and the user profile includes a first user profile stored at the first server and a second user profile at the second server, wherein the second user profile includes at least the first user profile, wherein the second user profile and the first user profile are associated with the same user, and the second server is connected to a remote monitoring interface (120), wherein the RPM system is further configured to:
- send by the first server (132) the physiological data with the recognition score to the second server (134),
- remove the generated physiological data of the second user profile based on the recognition score.

10. The RPM system of claims 7 and 9 or of claims 8 and 9, wherein the notification channel is included in the second user profile, and triggering is performed by the second server.

11. The RPM system of any of claims 1-10, further configured to:
- send to the physiological measurement device instructions to delete the generated physiological data and the generated recognition data.

12. The RPM system of any of claims 1-11 further comprising a remote monitoring interface (120), wherein the remote monitoring interface has a specific visual feature for the physiological data with a recognition score under a certainty threshold.

13. The RPM system of any of claims 1-12, wherein the recognition data are taken from the physiological data, for example the recognition data include at least one of weight data and impedance data.

14. A method of remotely monitoring patients, using a RPM system of any of claims 1-13, the method comprising:
- generating physiological data by the physiological measurement device,
- generating recognition data by the physiological measurement device, wherein the recognition data are associated with the physiological data,
- receiving by the server the generated recognition data and the generated physiological data, wherein the physiological data and the recognition data are attributed to the user profile of the server,
- computing a recognition score based at least one the generated recognition data, the recognition data being data used to determine whether the physiological data were generated by the user of the user profile,
- managing the generated physiological data based at least one the recognition score.

15. A body scale (110a) storing a user profile associated with a user, and configured to exchange data with a server, wherein every data generated by the body scale (110a) is systematically associated with the user profile of the server, wherein the body scale is configured to:
- generate physiological data,
- generate recognition data, associated with the physiological data,
- compute a recognition score based at least one the generated recognition data, the recognition data being data used to determine whether the physiological data were generated by the user of the user profile,
- send the recognition score and the physiological data to server,
- manage, at the body scale (110a) and/or the server, the generated physiological data based at least on the recognition score.
